# EUROPEAN PATENT APPLICATION

(11) **EP 2 243 483 A1**
(43) Date of publication of application: **27.10.2010**
(21) Application number: 10160506.1
(22) Date of filing: 20.04.2010
(51) Int. Cl.: A61K 31/4422, A61K 9/00, A61P 9/00, A61P 17/02

(54) **Amlodipine salt compositions for topical application**

(30) Priority: 21.04.2009 GB 0906868
(71) Applicant: Rabin, Bennie, Barnet, Hertfordshire EN5 4LB (GB)
(72) Inventor: Rabin, Bennie, Barnet, Hertfordshire EN5 4LB (GB)
(74) Representative: Jacob, Reuben Ellis

(57) **Abstract**

The present application relates to a topical composition comprising one or more amlodipine salts for the treatment of a circulatory condition, such as Raynaud's phenomenon or chilblains. The present application also relates to the use of one or more amlodipine salts to prepare a topical composition for the treatment of a circulatory condition.

## Description

This patent application relates to amlodipine salt compositions and more particularly to topical compositions comprising one or more amlodipine salts, such as amlodipine maleate for the treatment of circulatory conditions.

Two circulatory conditions are of particular interest, namely Raynaud's Phenomenon and chilblains.

Raynaud's Phenomenon (RP), also known as Raynaud's Syndrome, is a condition in which blood is prevented from reaching the extremities of the body, mainly the fingers and toes but sometimes the ears and the nose, on exposure to the cold or any slight change in temperature. RP affects the blood vessels (such as the arteries) that supply blood to the skin.

During a Raynaud's attack, the arteries narrow, thereby limiting blood circulation to the extremities. The effect is cold, white fingers with pins and needles and numbness, which eventually turn blue, then red, and are sometimes painful. The condition is described as being either primary, the commonest form, where there is no apparent cause for it, or secondary, where it is associated with an underlying disease.

The current experimental treatment is a nitroglycerin composition, usually in the form of a 0.9% cream. However, nitroglycerin is known to cause severe headaches to patients and to be unstable.

Chilblains (also known as pernio, perniosis or blain) can sometimes be a symptom of circulatory conditions such as Raynaud's Syndrome where the blood supply to the extremities is interrupted. Small lumps appear on the surface of the skin and can be itchy red and irritating.

Although they are very common, chilblains rarely cause any long-term problems and no treatment is normally required. However, where the symptoms persist, treatments include the use of a range of soothing creams and lotions (such as calamine lotion) that may ease the soreness until the symptoms disappear and steroid creams are also used to reduce itch and soreness.

Patients with persistent or bothersome symptoms of RP or chilblains may be helped by taking oral medications that dilate blood vessels. Such oral medications may include antagonists, such as nifedipine. These medications have a very slow onset and take on average around 4-6 hours to have an effect.

There is therefore a need for medication for the treatment of circulatory conditions such as RP or chilblains, which is stable, fast-acting and efficient. It is therefore an object of this invention to mitigate problems such as those described above.

According to a first aspect of the invention, there is provided a topical composition comprising one or more amlodipine salts for the treatment of a circulatory condition. Preferably, the condition is one that requires improved local blood flow. The amlodipine salt(s) may be selected from amlodipine maleate, amlodipine besilate and amlodipine mesylate, most preferably amlodipine maleate. Amlodipine salts such as amlodipine maleate are particularly beneficial in a topical composition for the treatment of RP and chilblains.

Amlodipine maleate is preferably present in the composition in an amount of from about 0.01 to about 5.00 wt%. More preferably, the composition comprises about 0.05 wt%, about 0.10 wt%, about 0.30 wt%, about 1.00 wt%, about 3.00 wt% or about 5.00 wt% amlodipine maleate. The composition may further comprise one or more surfactants (such as Poloxamer 407), solvent enhancers (such as propylene glycol, polyethylene glycol), thickeners (such as Carbopol 980) and/or permeation enhancers (carboxylic acids, water).

Preferably, the composition is in the form of a gel, ointment, cream, emollient, lotion, powder, solution, suspension, spray, paste, oil, foam, suppository or enema. Each dose of the composition may comprise from about 0.05 to about 0.10 g of amlodipine salt(s).

According to a second aspect of the invention, there is provided the use of one or more amlodipine salts to prepare a topical composition for the treatment of a circulatory condition.

Amlodipine maleate is a dihydropyridine calcium channel blocker that has vasodilating properties, i.e. it can widen blood vessels by relaxing smooth muscle cells within the vessel wall. Calcium channel blockers trigger changes in the resting membrane potential of the cell that affects the level of intracellular calcium through modulation of voltage sensitive calcium channels in the plasma membrane (so-called hyperpolarization mediated vasodilation).

Amlodipine maleate has been used in the treatment of certain types of angina, such as Prinzmetal angina when it is administered as a tablet.

Oral absorption of a therapeutic dose produces a peak concentration between 6 and 12 hours and absolute bioavailability is estimated to be between 64% and 90%. The administration of amlodipine maleate as a topical composition result in higher absorption rates than with tablets.

Studies show that the time required for therapeutic response when amlodipine maleate tablets are administered is initially 7 days and peaks at about 14 days. Approximately 93% of the circulating drug is bound to plasma protein in hypertensive patients. After 7 to 8 days of consecutive daily dosing, plasma steady state is achieved. When amlodipine maleate is administered as a topical composition, more uniform plasma levels are obtained and side effects are reduced due to maintenance of plasma levels up to the end of the dosing interval compared to a decline in plasma levels with conventional oral dosage forms.

About 90% of amlodipine maleate from a tablet is converted to inactive metabolites via hepatic metabolism. Metabolism is mostly through the CYP 3A4 isoenzyme. Amlodipine is moderately inhibited by CYP 1A2 isoenzyme with a weaker inhibition by 2A6, 2B6, 2C8/9, 2D6 and 3A4. It is thought that when amlodipine maleate is administered as a topical composition first pass metabolism can be minimised.

The mean terminal half-life of orally absorbed amlodipine is about 30-50 hours. Amlodipine is eliminated from the plasma by a biphasic mechanism. 10% of the parent compound and 60% of the metabolites are excreted in the urine. By contrast, when a topical composition is applied, the treatment may be terminated more easily, with residual amlodipine maleate being eliminated promptly from the system. It is also easier to adjust the dose of active component administered to the patient when amlodipine maleate is administered as a topical composition.

The composition is applied to the fingers and rubbed onto the affected areas, such as fingers, toes, nose and ears until it is at least partially absorbed by the skin.

Fluctuations (both inter- and intra-patients) in drug level and gastro-intestinal incompatibility can be minimised. In addition, the risks and inconveniences of oral treatment and of the varied conditions of absorption are avoided (e.g. pH changes, presence of enzymes, gastric emptying time and the like).

Also side effects will be less than those associated with the oral form of medication currently used for this condition because the drug in the topical form will not have to pass through the stomach and/or liver where the drug is broken down before reaching its intended site of action. Applying amlodipine maleate topically will limit the side effects seen with oral tablets, such as headaches, ankle swelling, facial flushing etc as applying it topically is avoids systemic absorption. Thus, physiological and pharmacological responses are improved.

It is thought that most users may not choose this compound to treat circulatory conditions because of the risk of lowering blood pressure in certain patients. However, amlodipine maleate is potent and more potent than most calcium channel blockers. Thus, the dosage required for treatment is minimal and thus side effects such as low blood pressure are minimal.

Another advantage observed in the treatment of circulatory conditions, is the reduction of the risk of infections and ulcers which are symptoms of conditions such as RP and chilblains. The topical composition improves the blood flow to and around that the area of application, thereby reducing the risk of infections and ulcers. The topical composition minimises or prevents further damage to the damaged nerve and lessens the risk of further infections and ulcers due to lack of blood flow.

The effects of topical compositions of amlodipine maleate are vastly superior to the treatments used for the conditions discussed above as it is a very potent vasodilator, and thus it brings more blood flow to an area that requires it, which is what these conditions require in order to be rectified. Most of the treatments currently available for these conditions are used to relieve pain and discomfort instead of clinically treating the patient.

Amlodipine maleate topical compositions are efficient in treating circulatory conditions such as RP and chilblains. Such compositions are convenient and easy to use so that patient compliance is notably improved.

The following Example is provided to illustrate absorption of a topical composition according to the invention, and with reference to the following Figures in which:

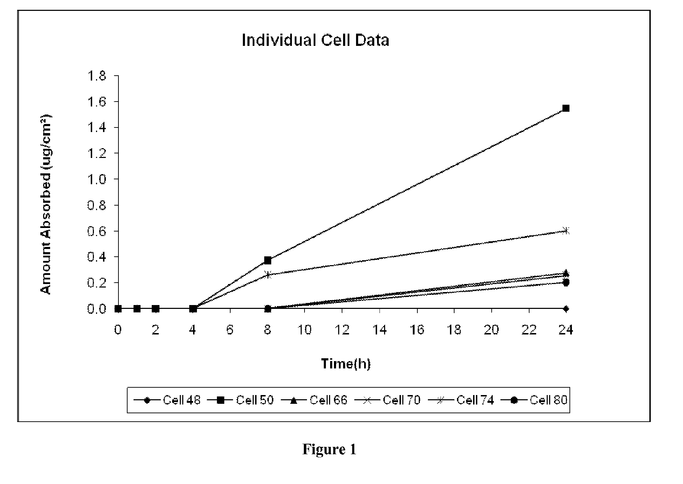

Figure 1 is a graph displaying individual cell data of amlodipine absorption from 3% w/v amlodipine maleate gel FO-0839 through pig epidermis; and

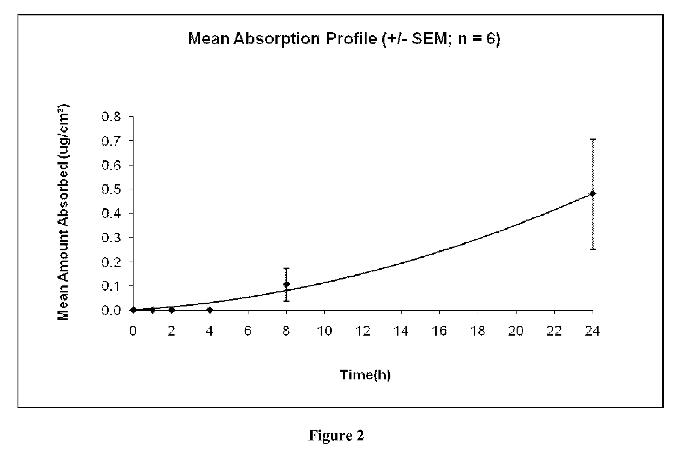

Figure 2 is a graph displaying the mean absorption profile of amlodipine absorption from 3% w/v amlodipine maleate gel FO-0839 through pig epidermis.

### Example

Initial tests of the *in vitro* absorption of amlodipine through pig epidermis over a 24 hour exposure period were conducted using four gels containing 0.3% w/v, 1% w/v, 3% w/v and 5% w/w amlodipine maleate. The best results were obtained using the 3% w/v gel and so this strength of gel was used in the detailed tests (and will be used in a clinical trial to be carried out in the University Hospital Aintree, Liverpool).

This Example was based upon the following guidelines:
OECD Test Guideline 428 (2004), Skin Absorption: *In Vitro* Method - a validated system for determining dermal absorption across human skin *in vitro*; and
OECD (Guidance Document No. 28 (2004), The Conduct of Skin Absorption Studies.

| | |
|---|---|
| Name: | Amlodipine maleate gel |
| DTL test substance reference number: | TS00100/008/001 |
| Source: | NuPharm Laboratories Ltd. |
| Batch number: | FO-0839 |
| Amlodipine content | 3.0% w/v |
| Physical state: | Gel |
| Storage conditions: | Ambient |

### Analytical techniques

Samples collected during this study were analysed by HPLC.

| High performance liquid chromatography (HPLC): | |
|---|---|
| Analysis was performed using the method detailed below. | |
| Equipment: | HPLC system = Agilent 1100 |
| Column: | Zorbax SB 300A, 5µm, 250 x 4.6 mm |
| Mobile phase: | Potassium dihydrogen phosphate 0.05 M, pH 7/Acetonitrile: 65/35% v/v |
| Flow rate: | 1 mL/min |
| Injection volume: | 20 µL |
| Column temperature: | Ambient |
| UV detector wavelength: | 240 nm |

The limit of detection for the receptor fluid using HPLC was set at 0.046 µg/mL (≡ 0.082 µg/cm²) and the limit of quantitation was set at 0.093 µg/mL (≡ 0.164 µg/cm²).

### Skin membranes

Ears from adult pigs were obtained from an abattoir. The ears were washed under running water and any visibly damaged ears rejected. The upper side of the remaining ears was clipped with animal clippers and whole skin (epidermis + dermis) was separated from the cartilage using a scalpel. The skin samples were immersed in water at 60 °C for 55-60 seconds and the epidermis teased away from the dermis.

### Assembly of diffusion cells

The type of static glass diffusion cell used in this study has an exposed membrane area of 2.54 cm2 and a volume of approximately 4.5 mL. Discs of approximately 3.3 cm diameter of prepared skin membrane were mounted, dermal side down, in diffusion cells held together with individually numbered clamps and placed in a water bath maintained at 32 °C ± 1 °C.

### Measurement of membrane integrity

Membrane integrity was determined by measurement of the electrical resistance across the skin membrane. Membranes with a measured resistance of <3 kΩ (Davies et al, 2004) were regarded as having a lower integrity than normal and not used for exposure to the test material.

### Selection of cells and dosing

Cells were selected such that each application was represented by six intact membranes from three different animals. The receptor chambers of the cells containing small magnetic stirrer bars were filled with a recorded volume of receptor fluid (50% ethanol in water). The 50% ethanol in water receptor fluid was chosen to ensure that the test substance could freely partition into the receptor fluid from the skin membrane and never reach a concentration that would limit its diffusion. A pre-treatment sample (250 µL) was taken from each receptor chamber for analysis by HPLC. An equal volume of fresh receptor fluid was added to each receptor chamber to replace the volume removed.

The gel was applied to the epidermal membranes by weight and spread with a glass rod. The cells were then placed in a water bath maintained at a temperature of 32 °C ± 1 °C. The applications were left unoccluded for the duration of the exposure period (24 hours).

Absorption of amlodipine from the 3% w/v gel (FO-0839) through pig epidermis was observed with a mean absorption rate of 0.021 µg/cm²/h during the 24 hour exposure period. A small amount of amlodipine was detected at 8 hours; the total amount of amlodipine absorbed at 24 hours was 0.481 µg/cm². Amlodipine from the 3% w/v gel was detectable in the receptor fluid after 8 hours.

## Claims

1. A topical composition comprising one or more amlodipine salts for the treatment of a circulatory condition.

2. The composition according to claim 1, wherein the circulatory condition requires improved local blood flow.

3. The composition according to claim 1 or 2, wherein the condition is Raynaud's Phenomenon.

4. The composition according to claim 1 or 2, wherein the condition is chilblains.

5. The composition according to any one of the preceding claims, wherein the amlodipine salt(s) is/are selected from amlodipine maleate, amlodipine besilate and amlodipine mesylate, preferably amlodipine maleate.

6. The composition according to any one of the preceding claims, wherein amlodipine maleate is present in an amount of from 0.01-5.00 wt %.

7. The composition according to any one of the preceding claims further comprising one or more of surfactants, solvent enhancers, thickeners and/or permeation enhancers.

8. The composition according to any one of the preceding claims, wherein the composition is in the form of a gel, ointment, cream, emollient, lotion, powder, solution, suspension, spray, paste, oil, foam, suppository or enema.

9. The composition according to any one of the preceding claims, wherein 0.05-0.10 g of amlodipine salt(s) is/are applied to a patient in need thereof per dose of the composition.

10. Use of one or more amlodipine salts to prepare a topical composition for the treatment of a circulatory condition.
